(19) Europäisches Patentamt
European Patent Office
Office européen des brevets

(11) Veröffentlichungsnummer: **0 269 837**
**A2**

# (12) EUROPÄISCHE PATENTANMELDUNG

(21) Anmeldenummer: 87115566.9

(22) Anmeldetag: 23.10.87

(51) Int. Cl.⁴: **C07D 207/456**, A01N 43/36, //C07D207/416

Die Bezeichnung der Erfindung wurde geändert (Richtlinien für die Prüfung im EPA, A-III, 7.3).

(30) Priorität: 04.11.86 DE 3637507

(43) Veröffentlichungstag der Anmeldung: 08.06.88 Patentblatt 88/23

(84) Benannte Vertragsstaaten: BE CH DE FR GB IT LI NL

(71) Anmelder: BAYER AG
Konzernverwaltung RP Patentabteilung
D-5090 Leverkusen 1 Bayerwerk(DE)

(72) Erfinder: Kühle, Engelbert, Dr.
von-Bodelschwingh-Strasse 42
D-5060 Bergisch-Gladbach 2(DE)
Erfinder: Adler, Alfons, Dr.
Hahnenweg 8
D-5000 Köln 80(DE)

## (54) Verfahren zur Herstellung von 3-Sulfenyl-maleinimiden und ihre Verwendung.

(57) Neues Verfahren zur Herstellung von teilsweise neuen 3-Sulfenylmaleinimiden der Formel (I)

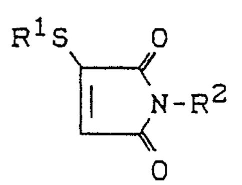

(I)

in welcher
$R^1$ und $R^2$ unabhängig voneinander für Alkyl, Cycloalkyl oder Aralkyl stehen, $R^2$ außerdem für Aryl steht, dadurch gekennzeichnet, daß man 3-Sulfenylsuccinimide der Formel (II)

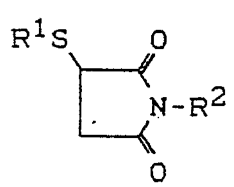

(II)

in welcher
$R^1$ und $R^2$ die oben angegebenen Bedeutungen haben,
mit Wasserstoffperoxid im Molverhältnis 1:1 in Gegenwart einer niederen Alkancarbonsäure bei Temperaturen zwischen 20°C und 120°C umsetzt, sowie die Verwendung einiger Stoffe als Fungizide und einige neue Stoffe.

EP 0 269 837 A2

## Neues Verfahren zur Herstellung von teilweise neuen 3-Sulfenylmaleinimiden und ihre Verwendung

Die vorliegende Erfindung betrifft ein neues Verfahren zur Herstellung von teilweise neuen 3-Sulfenylmaleinimiden und ihre Verwendung als Schädlingsbekämpfungsmittel.

Es ist bekannt, daß man 3-Sulfenylmaleinimide erhält, wenn man 3-Chlormaleinimide mit Mercaptanen gegebenenfalls in Form von Alkalisalzen gegebenenfalls in Gegenwart von Lösungsmitteln umsetzt (vgl. DD-Patent 76.122 und DOS 2 143 601). Das Verfahren weist den Nachteil auf, daß die zu verwendenden Ausgangsmaterialien - die 3-Chlormaleinimide - über zwei Stufen aus Maleinimiden und Chlor über die entsprechenden Dichlorbernsteinsäure-Derivate durch Clorwasserstoffabspaltung gewonnen werden.

Weiterhin ist bekannt, daß man 3-Sulfenylmaleinimide erhält, wenn mann Sulfenchloride mit Maleinimiden umsetzt. Die Ausgangsverbindungen - die Sulfenchloride -werden aus Mercaptanen durch Chlorierung gewonnen. Die Ausbeute der Endstufe liegt bei nur 42 % an einem noch verunreinigten Produkt (vgl. z.B. US 3.184.475 und US 3.224.936).

Es wurde gefunden, daß man die 3-Sulfenylmaleinimide der allgemeinen Formel (I)

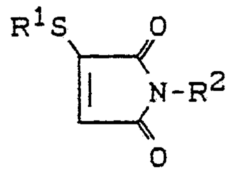

(I)

in welcher

$R^1$ für Alkyl, für gegebenenfalls ein-bis mehrfach substituiertes Cycloalkyl oder für gegebenenfalls ein-bis mehrfach substituiertes Aralkyl steht und

$R^2$ für gegebenenfalls ein-bis mehrfach substituiertes Alkyl, für gegebenenfalls ein-bis mehrfach substituiertes Cycloalkyl, für gegebenenfalls ein-bis mehrfach substituiertes Aryl oder für gegebenenfalls ein-bis mehrfach substituiertes Aralkyl steht,

nach einem neuen Verfahren erhält, wenn man 3-Sulfenylsuccinimide der allgemeinen Formel (II)

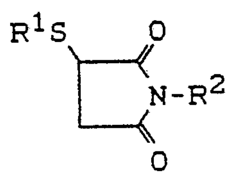

(II)

in welcher

$R^1$ and $R^2$ die oben angegebenen Bedeutungen haben,

mit Wasserstoffperoxid im Molverhältnis 1:1 in Gegenwart einer niederen Alkancarbonsäure als Lösungsmittel bei Temperaturen zwischen 20°C und 120°C umsetzt.

Vorzugsweise stehen in der Formel (I)

$R^1$ für geradkettiges oder verzweigtes Alkyl mit 1 bis 12 Kohlenstoffatomen, für gegebenenfalls ein-bis mehrfach, gleich oder verschieden substituiertes Cycloalkyl mit 5 bis 10 Kohlenstoffatomen oder für gegebenenfalls ein-bis mehrfach, gleich oder verschieden substituiertes Aralkyl mit 6 bis 10 Kohlenstoffatomen im Arylteil und gegebenenfalls 1 bis 4 Kohlenstoffatomen im Alkylteil, wobei als Substituenten im Aryl genannt seien: Halogen, Alkyl oder Alkoxy mit jeweils 1 bis 4 Kohlenstoffatomen, Alkylthio mit 1 bis 4 Kohlenstoffatomen, Halogenalkyl, Halogenalkoxy und Halogenalkylthio mit je 1 bis 3 Kohlenstoffatomen und 1 bis 5 Halogenatomen und Nitro;

$R^2$ für geradkettiges oder verzweigtes Alkyl mit 1 bis 12 Kohlenstoffatomen, für gegebenenfalls ein-bis mehrfach, gleich oder verschieden substituiertes Cycloalkyl mit 5 bis 10 Kohlenstoffatomen oder für gegebenenfalls ein-bis mehrfach, gleich oder verschieden substituiertes Aralkyl mit 6 bis 10 Kohlenstoffatomen im Arylteil und 1 bis 4 Kohlenstoffatomen im Alkylteil oder für gegebenenfalls ein-oder mehrfach, gleich oder verschieden substituiertes Aryl mit 6 bis 10 Kohlenstoffatomen, wobei als Substituenten im Arylteil des Aralkyls oder im Aryl selbst genannt seien: Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl, Halogenalkoxy und Halogenalkylthio mit je 1 bis 3 Kohlenstoffatomen und 1 bis 5 Halogenatomen und Nitro.

Besonders bevorzugt stehen in der Formel (I)

$R^1$ für geradkettiges oder verzweigtes Alkyl mit 1 bis 8 Kohlenstoffatomen, für gegebenenfalls ein-bis vierfach durch alkyl mit 1 bis 4 Kohlenstoffatomen substituiertes Cycloalkyl mit 5 bis 8 Kohlenstoffatomen oder für gegebenenfalls ein-bis fünffach, gleich oder verschieden substituiertes Phenylalkyl mit 1 bis 3 Kohlenstoffatomen im Alkylteil, wobei als Substituenten im Phenyl genannt seien: Fluor, Chlor, Alkyl mit 1 bis 3 Kohlenstoffatomen, Alkoxy mit 1 bis 3 Kohlenstoffatomen, Alkylthio mit 1 bis 3 Kohlenstoffatomen, Halogenalkyl, Halogenalkoxy und Halogenalkylthio mit jeweils 1 oder 2 Kohlenstoffatomen und 1 bis 3 gleichen oder verschiedenen Halogenatomen, wie Fluor und Chlor, und Nitro und

$R^2$ für geradkettiges oder verzweigtes Alkyl mit 1 bis 8 Kohlenstoffatomen, für gegebenenfalls ein-bis vierfach durch Alkyl mit 1 bis 4 Kohlenstoffatomen substituiertes Cycloalkyl mit 5 bis 8 Kohlenstoffatomen oder für gegebenenfalls ein-bis fünffach, gleich oder verschieden substituiertes Phenylalkyl mit 1 bis 3 Kohlenstoffatomen im Alkylteil oder für ein-bis fünffach, gleich oder verschieden substituiertes Phenyl, wobei als Substituenten des Phenyls und im Phenyl des Phenylalkyls genannt seien: Fluor, Chlor, Alkyl mit 1 bis 3 Kohlenstoffatomen, Alkoxy mit 1 bis 3 Kohlenstoffatomen, Alkylthio mit 1 bis 3 Kohlenstoffatomen, Halogenalkyl, Halogenalkoxy und Halogenalkylthio mit jeweils 1 oder 2 Kohlenstoffatomen und 1 bis 3 gleichen oder verschiedenenen Halogenatomen, wie Fluor und Chlor, und Nitro.

Ganz besonders bevorzugt stehen in der Formel (I)

$R^1$ für geradkettiges oder verzweigtes Alkyl mit 1 bis 5 Kohlenstoffatomen oder für Cyclohexyl und

$R^2$ für geradkettiges oder verzweigtes Alkyl mit 1 bis 5 Kohlenstoffatomen, für Cyclohexyl, für gegebenenfalls ein-bis dreifach, gleich oder verschieden jeweils substituiertes Phenyl, Benzyl oder Phenethyl, wobei als Substituenten genannt seien: Chlor, Methyl, Ethyl, Trifluormethyl, Trifluormethoxy, Trifluormethylthio, Chlordifluormethoxy, 2-Chlor-1,1-difluorethoxy und Nitro.

Es ist als ausgesprochen überraschend zu bezeichnen, daß gemäß der erfindungsgemäßen Umsetzung von 3-Sulfenylsuccinimiden der Formel (II) mit 1 Mol Wasserstoffperoxid die teilweise neuen Verbindungen der Formel (I) in so guten Ausbeuten und hoher Reinheit erhalten werden, weil man im Hinblick auf den Stand der Technik erwarten mußte, daß die Reaktion zu einem Gemisch verschiedener Produkte führt, da es seit langem bekannt ist, daß 3-Sulfenylsuccinimide mit überschüssigem Wasserstoffperoxid zu 3-Sulfonylsuccinimiden oxidiert werden (vgl. z.B. DOS 2.143.601 und DOS 3.026.755).

Das erfindungsgemäße Verfahren weist eine Reihe von Vorteilen auf. So werden die Endprodukte in einer einfachen Einstufenreaktion in hoher Reinheit und in guten Ausbeuten erhalten, wobei die benötigten Ausgangsverbindungen der Formel (II) ebenfalls in einfacher und glatter Weise in einem Einstufenprozeß hergestellt werden können.

Verwendet man 3-n-Butylsulfenyl-1-phenyl-succinimid als Ausgangsverbindung und setzt diese mit 1 Mol 35 %igem Wasserstoffperoxid um, so kann der Verlauf des erfindungsgemäßen Verfahrens durch das folgende Formelschema veranschaulicht werden:

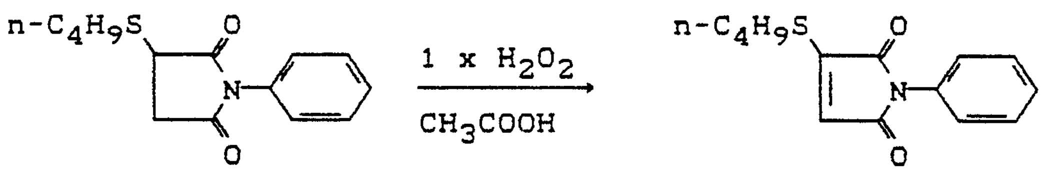


Die bei dem erfindungsgemäßen Verfahren als Ausgangsstoffe benötigten 3-Sulfenylsuccinimide sind durch die Formel (II) allgemein definiert. In dieser Formel haben $R^1$ und $R^2$ diejenigen Bedeutungen, die bereits im Zusammenhang mit der Beschreibung des erfindungsgemäßen Verfahrens für die Verbindungen der Formel (I) für diese Reste genannt wurden.

Als Beispiele für 3-Sulfenylsuccinimide seien folgende Verbindungen erwähnt:

3-Methylsulfenylsuccinimid am Stickstoff substituiert durch Methyl, Butyl, tert. Butyl, Cyclopentyl, Cyclohexyl, Benzyl, 4-Trifluormethylbenzyl, 4-Trifluormethoxybenzyl, Phenyl, 4-Tolyl-, 4-Chlorphenyl, 3-Nitrophenyl, 3,4-Dichlorphenyl, 3-Trifluormethylphenyl, 4-Trifluormethylphenyl oder 4-Trifluormethylthiophenyl.

3-Isopropylsulfenylsuccinimid am Stickstoff substituiert durch Isopropyl, Isooctyl, Dodecyl, 3,3,5-Trimethylcyclohexyl, 2-Phenethyl, 2-Tolyl, 2,4-Dichlorphenyl, 2,6-Dimethylphenyl, 3-Trifluormethylphenyl, 4-(2-Chlor-1,2,2-trifluorethoxy)-phenyl.

3-n-Butylsulfenylsuccinimid am Stickstoff substituiert durch Methyl, Isobutyl, Cyclohexyl, 4-Chlorbenzyl, Phenyl, 2-Chlorphenyl , 2-Nitrophenyl, 2,6-Diethylphenyl.

3-Cyclohexylsulfenylsuccinimid am Stickstoff substituiert durch Methyl, Neopentyl, Cyclohexyl, Phenyl, 2-Trifluormethoxyphenyl.

Die 3-Sulfenylsuccinimide sind teilweise bekannt.

Sie können z.B. hergestellt werden

1) durch Addition von Thiolen an N-substituierte Maleinimide (vgl. DOS 3 026 755) oder

2) durch Cyclisierung eines 3-Sulfenylbernsteinsäuremonoamids unter Dehydratisierung (vgl. DOS 2 143 601). Außerdem sei die Herstellungsbeispiele verwiesen.

Das ebenfalls benötigte Wasserstoffperoxid ist ein großtechnisch herstellbares, käufliches Produkt.

Als Verdünnungs-oder Lösungsmittel für das erfindungsgemäße Verfahren kommen niedere Alkancarbonsäuren infrage. Hierzu gehören vorzugsweise Ameisensäure, Essigsäure und Propionsäure, die gegebenenfalls auch Wasser enthalten können.

Die Umsetzung wird bei Temperaturen zwischen 20°C und 120°C vorzugsweise zwischen 80°C und 120°C vorgenommen.

Die Umsetzung wird normalerweise bei Normaldruck ausgeführt.

Die Ausgangsverbindungen der Formel (II) werden mit dem Wasserstoffperoxid im Molverhältnis 1:1 umgesetzt.

Bei der Durchführung des erfindungsgemäßen Verfahrens legt man im allgemeinen das 3-Sulfenylsuccinimid in der niederen Alkansäure vor und tropft im angegebenen Temperaturbereich das Wasserstoffperioxid zu. Zur Vervollständigung der Reaktion wird vorzugsweise noch einige Minuten nacherhitzt, abgekühlt und durch Zugabe von Wasser das gewünschte Produkt ausgefällt, das nach dem Absaugen und Trocknen meist schon in hoher Reinheit vorliegt, so daß sich ein Umkristallisieren erübrigt.

Die nach dem erfindungsgemäßen Verfahren herstellbaren Verbindungen und ihre Verwendung sind teilweise bekannt.

So is bekannt, daß 3-Sulfenylmaleinimide, wie das 3-Ethylsulfenyl-1-phenyl-maleinimid, als Blattfungizide, als Saatgutbeizmittel und zum Schutz von verschiedenen Materialien in Betracht kommen (vgl. DD-Patent 76.122). Ferner ist bekannt, daß 3-Phenylsulfenyl-1-alkyl-maleinimide eine Wirkung gegen Bakterien und Pilze besitzen (vgl. US 3.224.936). Auch 3-Alkylsulfenyl-bzw. 3-Phenylsulfenyl-1-phenylmaleinimide sind zur Verwendung im Materialschutz bekannt (vgl. Jap. Patent 53 032 119).

Es wurde gefunden, daß die Verwendung der Verbindungen der Formel (IA)

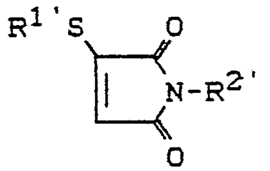

(IA)

in welcher

$R^{1'}$ für Alkyl, für gegebenenfalls ein-bis mehrfach substituiertes Cycloalkyl oder für gegebenenfalls ein-bis mehrfach substituiertes Aralkyl steht und

$R^{2'}$ für gegebenenfalls ein-bis mehrfach substituiertes Alkyl, für gegebenenfalls ein-bis mehrfach substituiertes Cycloalkyl, für gegebenenfalls ein-bis mehrfach substituiertes Aryl oder für gegebenen falls ein-bis mehrfach substituiertes Aralkyl steht, ausgenommen die Verbindung 3-Ethylsulfenyl-1-phenylmaleinimid, als Fungizide im Pflanzenschutz neu und ein Teil der Erfindung ist.

Überraschenderweise zeigen die 3-Sulfenylmaleinimide der Formel (IA) eine bessere funigzide Wirkung als die aus dem Stand der Technik bekannten strukturell und wirkungsmäßig ähnlichen Verbindungen.

Vorzugsweise stehen in der Formel (IA)

$R^{1'}$ für geradkettiges oder verzweigtes Alkyl mit 1 bis 12 Kohlenstoffatomen, für gegebenenfalls ein-bis vierfach durch Alkyl mit 1 bis 4 Kohlenstoffatomen substituiertes Cycloalkyl mit 5 bis 10 Kohlenstoffatomen oder für gegebenenfalls ein-bis mehrfach, gleich oder verschieden substituiertes Aralkyl mit 6 bis 10 Kohlenstoffatomen im Arylteil und 1 bis 4 Kohlenstoffatomen im Alkylteil, wobei als Substituenten im Aryl genannt seien: Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl, Halogenalkoxy und Halogenalkylthio mit je 1 bis 3 Kohlenstoffatomen und 1 bis 5 Halogenatomen und Nitro;

$R^{2'}$ für geradkettiges oder verzweigtes Alkyl mit 1 bis 12 Kohlenstoffatomen, für gegebenenfalls ein-bis vierfach durch Alkyl mit 1 bis 4 Kohlenstoffatomen substituiertes Cycloalkyl mit 5 bis 10 Kohlenstoff atomen oder für gegebenenfalls ein-bis mehrfach, gleich oder verschieden substituiertes Aralkyl mit 6 bis 10 Kohlenstoffatomen im Arylteil und 1 bis 4 Kohlenstoffatomen im Alkylteil oder für gegebenenfalls ein-oder mehrfach, gleich oder verschieden substituiertes Aryl mit 6 bis 10 Kohlenstoffatomen, wobei als Substituenten im Arylteil des Aralkyls oder im Aryl selbst genannt seien: Halogen, Alkyl, mit 1 bis 4 Kohlenstoffatomen, Alkoxy mit 1 bis 4 Kohlenstoffatomen, Alkylthio mit 1 bis 4 Kohlenstoffatomen, Halogenalkyl, Halogenalkoxy und Halogenalkylthio mit je 1 bis 3 Kohlenstoffatomen und 1 bis 5 Halogenatomen und Nitro, ausgenommen ist die Verbindung 3-Ethylsulfenyl-1-phenylmaleinimid.

Besonders bevorzugt stehen in der Formel (IA)

$R^{1'}$ für geradkettiges oder verzweigtes Alkyl mit 1 bis 8 Kohlenstoffatomen, für Cycloalkyl mit 5 bis 8 Kohlenstoffatomen oder für gegebenenfalls ein-bis fünffach, gleich oder verschieden substituiertes Phenylalkyl mit 1 bis 3 Kohlenstoffatomen im Alkylteil, wobei als Substituenten genannt seien: Fluor, Chlor, Alkyl mit 1 bis 3 Kohlenstoffatomen, Alkoxy mit 1 bis 3 Kohlenstoffatomen, Alkylthio mit 1 bis 3 Kohlenstoffatomen, Halogenalkyl, Halogenalkoxy und Halgenalkylthio mit jeweils 1 oder 2 Kohlenstoffatomen und 1 bis 3 gleichen oder verschiedenen Halogenatomen, wie Fluor und Chlor, und Nitro und

$R^{2'}$ für geradkettiges oder verzweigtes Alkyl mit 1 bis 8 Kohlenstoffatomen, für Cycloalkyl mit 5 bis 8 Kohlenstoffatomen oder für gegebenenfalls ein-bis fünffach, gleich oder verschieden substituiertes Phenylalkyl mit 1 bis 3 Kohlenstoffatomen im Alkylteil oder für ein-bis fünffach, gleich oder verschieden substituiertes Phenyl, wobei als Substituenten des Phenyls und im Phenyl des Phenylalkyls gennant seien: Fluor, Chlor, Alkyl oder Alkoxy mit jeweils 1 bis 3 Kohlenstoffatomen, Alkylthio mit 1 bis 3 Kohlenstoffatomen, Halogenalkyl, Halogenalkoxy und Halogenalkylthio mit jeweils 1 oder 2 Kohlenstoffatomen und 1 bis 3 gleichen oder verschiedenen Halogenatomen, wie Fluor und Chlor, und Nitro; ausgenommen die Verbindung 3-Ethylsulfenyl-1-phenylmaleinimid.

Ganz besonders bevorzugt stehen in der Formel (1A)

$R^{1'}$ für geradkettiges oder verzweigtes Alkyl mit 1 bis 5 Kohlenstoffatomen oder für Cyclohexyl und

$R^{2'}$ für geradkettiges oder verzweigtes Alkyl mit 1 bis 5 Kohlenstoffatomen, für Cyclohexyl, für gegebenenfalls ein-bis dreifach, gleich oder verschieden substituiertes Phenyl, Benzyl oder Phenethyl, wobei als Substituenten genannt seien: Chlor, Methyl, Ethyl, Trifluormethyl, Trifluormethoxy, Trifluormethylthio, Chlordifluormethoxy, 2-Chlor-1,1-difluorethoxy und Nitro; ausgenommen die Verbindung 3-Ethylsulfenyl-1-phenylmaleinimid.

Es wurde weiterhin gefunden, daß die nach dem erfindungsgemäßen Verfahren erhältlichen 3-Sulfenylmaleinimide der Formel (IB)

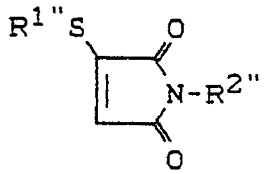

(IB)

in welcher

$R^{1''}$ für Alkyl, für gegebenenfalls substituiertes Cycloalkyl, oder für gegebenenfalls substituiertes Aralkyl steht und

$R^{2''}$ für gegebenenfalls substituiertes Cycloalkyl, für gegebenenfalls substituiertes Aralkyl oder für substituiertes Aryl steht, ausgenommen die Verbindungen, in denen $R^{1''}$ für Alkyl und $R^{2''}$ für unsubstituiertes oder durch Halogen, Alkyl, oder Alkoxy substituiertes Phenyl steht,

neu sind.

Vorzugsweise stehen in der Formel (IB)

$R^{1''}$ für geradkettiges oder verzweigtes Alkyl mit 1 bis 12 Kohlenstoffatomen, für gegebenenfalls ein-bis vierfach durch Alkyl mit 1 bis 4 Kohlenstoffatomen substituiertes Cycloalkyl mit 5 bis 10 Kohlenstoffatomen oder für gegebenenfalls ein-bis mehrfach, gleich oder verschieden substituiertes Aralkyl mit 6 bis 10 Kohlenstoffatomen im Arylteil und 1 bis 4 Kohlenstoffatomen im Alkylteil, wobei als Substituenten im Aryl genannt seien: Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl, Halogenalkoxy und Halogenalkylthio mit je 1 bis 3 Kohlenstoffatomen und 1 bis 5 Halogenatomen und Nitro;

$R^{2''}$ für gegebenenfalls ein-bis vierfach durch Alkyl mit 1 bis 4 Kohlenstoffatomen substituiertes Cycloalkyl mit 5 bis 10 Kohlenstoffatomen oder für gegebenenfalls ein-bis mehrfach, gleich oder verschieden substituiertes Aralkyl mit 6 bis 10 Kohlenstoffatomen im Arylteil und 1 bis 4 Kohlenstoffatomen im Alkylteil oder für gegebenenfalls ein-oder mehrfach, gleich oder verschieden substituiertes Aryl mit 6 bis 10 Kohlenstoffatomen, wobei als Substituenten im Arylteil des Aralkyls oder im Aryl selbst genannt seien: Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl, Halogenalkoxy und Halogenalkylthio mit je 1 bis 3 Kohlenstoffatomen und 1 bis 5 Halogenatomen und Nitro; ausgenommen die Verbindungen, in denen $R^{1''}$ für Alkyl und $R^{2''}$ für

unsubstituiertes oder durch Halogen, Alkyl oder Alkoxy substituiertes Phenyl steht.

Besonders bevorzugt stehen in der Formel (IB)

$R^{1''}$ für geradkettiges oder verzweigtes Alkyl mit 1 bis 8 Kohlenstoffatomen, für Cycloalkyl mit 5 bis 8 Kohlenstoffatomen oder für gegebenenfalls ein-bis fünffach, gleich oder verschieden substituiertes Phenylalkyl mit 1 bis 3 Kohlenstoffatomen im Alkylteil, wobei als Substituenten im Phenyl genannt seien: Fluor, Chlor, Alkyl mit 1 bis 3 Kohlenstoffatomen, Alkoxy mit 1 bis 3 Kohlenstoffatomen, Alkylthio mit 1 bis 3 Kohlenstoffatomen, Halogenalkyl, Halogenalkoxy und Halogenalkylthio mit jeweils 1 oder 2 Kohlenstoffatomen und 1 bis 3 gleichen oder verschiedenen Halogenatomen, wie Fluor und Chlor, und Nitro, und

$R^{2''}$ für Cycloalkyl mit 5 bis 8 Kohlenstoffatomen oder für gegebenenfalls ein-bis fünffach, gleich oder verschieden substituiertes Phenylalkyl mit 1 bis 3 Kohlenstoffatomen im Alkylteil oder für ein-bis fünffach, gleich oder verschieden substituiertes Phenyl, wobei als Substituenten des Phenyl und im Phenyl des Phenylalkyls genannt seien: Fluor, Chlor, Alkyl mit 1 bis 3 Kohlenstoffatomen, Alkoxy mit 1 bis 3 Kohlenstoffatomen, Alkylthio mit 1 bis 2 Kohlenstoffatomen, Halogenalkyl, Halogenalkoxy und Halogenalkylthio mit jeweils 1 oder 2 Kohlenstoffatomen und 1 bis 3 gleichen oder verschiedenen Halogenatomen, wie Fluor und Chlor, und Nitro; ausgenommen die Verbindungen, in denen $R^{1''}$ für Alkyl und $R^{2''}$ für unsubstituiertes oder durch Halogen, Alkyl oder Alkoxy sustituiertes Phenyl steht.

Ganz besonders bevorzugt stehen in der Formel (IB)

$R^{1''}$ für geradkettiges oder verzweigtes Alkyl mit 1 bis 5 Kohlenstoffatomen oder für Cyclohexyl und

$R^{2''}$ für Cyclohexyl, für gegebenenfalls ein-bis dreifach, gleich oder verschieden substituiertes Benzyl oder Phenethyl oder für substituiertes Phenyl, wobei als Substituenten genannt seien: Chlor, Methyl, Ethyl, Trifluormethyl, Trifluormethoxy, Trifluormethylthio, Chlordifluormethoxy, 2-Chlor-1,1-difluorethoxy und Nitro; ausgenommen die Verbindungen, in denen $R^{1''}$ für Alkyl und $R^{2''}$ für durch Halogen, Alkyl oder Alkoxy substituiertes Phenyl steht.

Die nach dem erfindungsgemäßen Verfahren herstellbaren Verbindungen der Formel (IA) und (IB) weisen gute biologische Wirkung auf und können zur Bekämpfung von Schädlingen eingesetzt werden. Die Wirkstoffe sind für den Gebrauch z.B. als Pflanzenschutzmittel geeignet, vor allem als Fungizide.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum;
Phytophthora-Arten, wie Beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz-und Saatgut, und des Bodens.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsufoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus, anorganischen und organischen Mehlen sowie Granulate aus organischem Material wie Sägemehl, Kokosnußschalen, Maiskolben und Tabakstengel. Als Emulgier-und/oder -schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide, Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von0,00001 bis 0,1 Gew.-% vorzugsweise von 0,0001 bis 0,02 %, am Wirkungsort erforderlich.

<u>Herstellungsbeispiele</u>

Beispiel 1

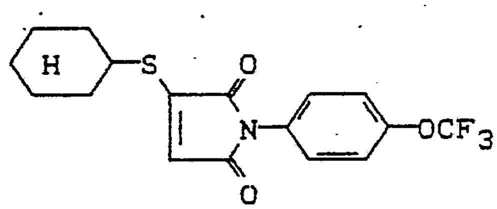

8 g (0,021 Mol) 3-Cyclohexylsulfenyl-1-(4-trifluormethoxyphenyl)-succinimid werden in 100 ml Essigsäure gelöst. Bei 90 bis 100°C werden 2,1 ml (0,021 Mol) 35 %iges wäßriges Wasserstoffperoxid zugetropft. Man erhitzt 10 Minuten lang zum Sieden, kühlt ab und setzt 200 ml Wasser zu. Hierbei fällt das 3-Cyclohexylsulfenyl-1-(4-trifluormethoxyphenyl)-maleinimid aus. Nach dem Absaugen und Trocknen erhält man 4 g (50 % der Theorie) an 3-Cyclohexylsulfenyl-1-(4-trifluormethoxyphenyl)-maleinimid mit einem Schmelzpunkt von 128 bis 132°C.

In ähnlicher Weise erhält man folgende 3-Sulfenylmaleinimide der Formel (I)

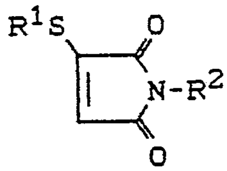

(I)

| Nr. | $R^1$ | $R^2$ | Ausbeute [% der Theorie] | Physik. Daten [Schmelzpunkt °C; Brechungsindex $n_D^{20}$] |
|---|---|---|---|---|
| 2 | $i-C_3H_7$ | $4-CF_3O-C_6H_4-$ | 67 | 112-114 |
| 3 | " | $4-CF_3S-C_6H_4-$ | 56 | 126-128 |
| 4 | " | $4-CH_3-C_6H_4-$ | 65 | 110-112 |
| 5 | " | $2,4-Cl_2C_6H_3-$ | 98 | 1,5072 |
| 6 | " | $2-NO_2-C_6H_4-$ | 97 | 1,5088 |
| 7 | " | $2-Cl-C_6H_4-$ | 70 | 1,5704 |
| 8 | " | $2-CH_3-C_6H_4-$ | 95 | 1,5651 |
| 9 | " | $3-CH_3-C_6H_4-$ | 98 | 1,5063 |
| 10 | " | $C_6H_5-CH_2-$ | - | 71- 73 |
| 11 | " | $3-CF_3-C_6H_4-CH_2$ | - | 1,5302 |
| 12 | " | $3-Cl, 4-CF_3-C_6H_3-CH_2-$ | - | 1,5398 |
| 13 | $n-C_4H_9$ | $2,6-(C_2H_5)_2-C_6H_3-$ | 98 | 1,5464 |
| 14 | " | $4-CF_3O-C_6H_4-$ | 86 | 101-105 |
| 15 | " | $3,4-Cl_2-C_6H_3-$ | 83 | 105-107 |
| 16 | " | $2,4-Cl_2-C_6H_3-$ | 98 | 1,5389 |
| 17 | " | $2-Cl-C_6H_4-$ | 82 | 1,5631 |
| 18 | " | $C_6H_5-CH_2-$ | - | 77- 80 |
| 19 | " | $3-CF_3-C_6H_4-CH_2-$ | - | 1,5307 |
| 20 | $C_6H_{11}$ | $4-CF_3-C_6H_4-$ | 41 | 145-147 |
| 21 | " | $4-CH_3-C_6H_4-$ | 41 | 177-179 |
| 22 | " | $3-ClCH_2-CF_2O-C_6H_4-$ | 86 | 1,5590 |
| 23 | " | $2-CF_3O-C_6H_4-$ | 80 | 1,5363 |
| 24 | " | $4-CF_3O-C_6H_4-$ | 50 | 132 |
| 25 | " | $4-CF_2ClO-C_6H_4-$ | 67 | 117-121 |
| 26 | " | $3-Cl, 4-CF_3O-C_6H_4-$ | 40 | 132-134 |
| 27 | " | $2,4-(CF_3)_2-C_6H_3-$ | 94 | 1,5130 |
| 28 | " | $3-CF_3O-C_6H_4-$ | 72 | 95- 97 |
| 29 | " | $2-CF_3-C_6H_4-$ | 30 | 1,5054 |
| 30 | $i-C_3H_7$ | $C_6H_{11}$ | 55 | 85 |
| 31 | " | $i-C_4H_9-$ | 32 | 33 |

Herstellung der Ausgangsstoffe der Formel (II)

Beispiel A1

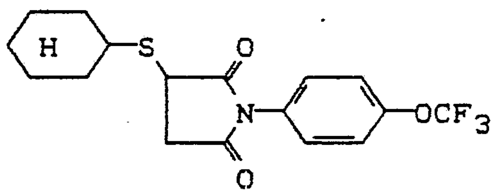

10,7 g (0,05 Mol) 3-Cyclohexylsulfenylbernsteinsäureanhydrid werden in 50 ml Toluol gelöst und mit der Lösung aus 8,9 g (0,05 Mol) 4-Trifluormethoxyanilin in 50 ml Toluol tropfenweise versetzt. Die Temperatur steigt hierbei aus 42°C an. Man kühlt ab, setzt etwas Petrolether zu und saugt die entstandene Amidsäure (Fp. 137 bis 139°C; Ausbeute 16 g) ab.

11 g dieser Amidsäure werden in 100 ml Xylol unter Zusatz von 1 ml konz. Schwefelsäure 1 Stunde lang am Wasserabscheider rückfließend erhitzt. Nach dem Erkalten setzt man 250 ml Wasser zu, trennt von wenig Unlöslichem, trocknet die Xylollösung und destilliert das Xylol im Vakuum ab. Man erhält 10 g 3-Cyclohexylsulfenyl-1-(4-trifluormethoxyphenyl)-succinimid mit einem Schmelzpunkt von 125 bis 127°C.

In ähnlicher Weise werden die nachfolgenden Verbindungen der Formel (II) hergestellt:

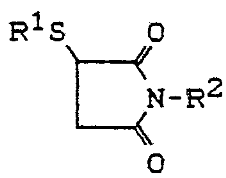

(II)

| Nr. | $R^1$ | $R^2$ | Physik. Daten [Schmelzpunkt °C; Brechungsindex $n_D^{20}$] |
|---|---|---|---|
| A2 | $i-C_3H_7$ | $4-CF_3-C_6H_4-$ | 122-123 |
| A3 | " | $4-CF_3-S-C_6H_4-$ | 95- 96 |
| A4 | " | $4-CH_3-C_6H_4-$ | 105-107 |
| A5 | " | $2,4-Cl_2C_6H_3-$ | 1,5086 |
| A6 | " | $2-NO_2-C_6H_4-$ | 1,5084 |
| A7 | " | $2-Cl-C_6H_4-$ | 1,5047 |
| A8 | " | $2-CH_3-C_6H_4-$ | 1,5056 |
| A9 | " | $3-CH_3-C_6H_4-$ | 60- 62 |
| A10 | " | $C_6H_5-CH_2-$ | 1,5534 |
| A11 | " | $3-CF_3-C_6H_4-CH_2$ | 1,5120 |
| A12 | " | $3-Cl,\ 4-CF_3-C_6H_3-CH_2$ | 1,5398 |
| A13 | $n-C_4H_9-$ | $2,6-(C_2H_5)_2-C_6H_3-$ | 50- 51 |
| A14 | " | $4-CF_3O-C_6H_4-$ | 69- 73 |
| A15 | " | $3,4-Cl_2-C_6H_3-$ | 77- 80 |
| A16 | " | $2,4-Cl_2-C_6H_3-$ | 1,5725 |
| A17 | " | $2-Cl-C_6H_4-$ | 1,5635 |
| A18 | " | $C_6H_5-CH_2$ | 1,5477 |
| A19 | " | $3-CF_3-C_6H_4-CH_2$ | 1,5105 |
| A20 | $C_6H_{11}-$ | $4-CF_3-C_6H_4$ | 144-145 |
| A21 | " | $4-CH_3-C_6H_4$ | 112-114 |
| A22 | " | $3-Cl-CH_2-CF_2O-C_6H_4$ | 1,5260 |
| A23 | " | $2-CF_3O-C_6H_4$ | 1,5260 |
| A24 | " | $4-CF_3O-C_6H_4$ | 125-127 |
| A25 | " | $4-CF_2ClO-C_6H_4$ | 111-113 |
| A26 | " | $3-Cl,\ 4-CF_3O-C_6H_3$ | 76- 78 |
| A27 | " | $2,4-(CF_3)_2-C_6H_3$ | 1,5143 |
| A28 | " | $3-CF_3O-C_6H_4$ | 57- 59 |
| A29 | " | $2-CF_3-C_6H_4$ | 1,5345 |
| A30 | $i-C_3H_7$ | $C_6H_{11}$ | ölig IR 1.685 cm |
| A31 | " | $i-C_4H_9$ | ölig 1.690 cm |

## Ansprüche

1. Verfahren zur Herstellung von 3-Sulfenylmaleinimiden der Formel (I)

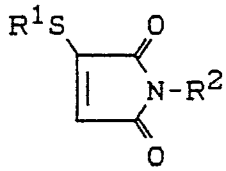

(I)

in welcher
$R^1$ für Alkyl, für gegebenenfalls ein-bis mehrfach substituiertes Cycloalkyl oder für gegebebenfalls ein-bis mehrfach substituiertes Aralkyl steht und
$R^2$ für gegebenenfalls ein-bis mehrfach substituiertes Alkyl, für gegebenenfalls ein-bis mehrfach substituiertes Cycloalkyl, für gegebenenfalls ein-bis mehrfach substituiertes Aryl oder für gegebenenfalls ein-bis mehrfach substituiertes Aralkyl steht,
dadurch gekennzeichnet, daß man 3-Sulfenylsuccinimide der allgemeinen Formel (II)

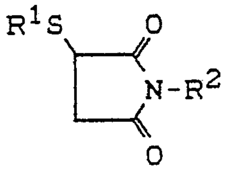

(II)

in welcher
$R^1$ und $R^2$ die oben angegebenen Bedeutungen haben,
mit Wasserstoffperoxid im Molverhältnis 1:1 in Gegenwart einer niederen Alkancarbonsäure einsetzt.

2. Verfahren gemäß Anspruch 1, worin in der Formel (I)
$R^1$ für geradkettiges oder verzweigtes Alkyl mit 1 bis 12 Kohlenstoffatomen, für gegebenenfalls ein-bis mehrfach, gleich oder verschieden substituiertes Cycloalkyl mit 5 bis 10 Kohlenstoffatomen oder für gegebenenfalls ein-bis mehrfach, gleich oder verschieden substituiertes Aralkyl mit 6 bis 10 Kohlenstoffatomen im Arylteil und gegebenenfalls 1 bis 4 Kohlenstoffatomen im Alkylteil steht, wobei als Substituenten im Aryl genannt seien: Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl, Halogenalkoxy und Halogenalkylthio mit je 1 bis 3 Kohlenstoffatomen und 1 bis 5 Halogenatomen und Nitro;
$R^2$ für geradkettiges oder verzweigtes Alkyl mit 1 bis 12 Kohlenstoffatomen, für gegebenenfalls ein-bis mehrfach, gleich oder verschieden substituiertes Cycloalkyl mit 5 bis 10 Kohlen stoffatomen oder für gegebenenfalls ein-bis mehrfach, gleich oder verschieden substituiertes Aralkyl mit 6 bis 10 Kohlenstoffatomen im Arylteil und 1 bis 4 Kohlenstoffatomen im Alkylteil oder für gegebenenfalls ein-oder mehrfach, gleich oder verschieden substituiertes Aryl mit 6 bis 10 Kohlenstoffatomen steht, wobei als Substituenten im Arylteil des Aralkyls oder im Aryl selbst genannt seien: Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl, Halogenalkoxy und Halogenalkylthio mit je 1 bis 3 Kohlenstoffatomen und 1 bis 5 Halogenatomen und Nitro.

3. Verfahren gemäß Anspruch 1, worin die niedere Alkancarbonsäure Ameisensäure, Essigsäure oder Propionsäure ist.

4.Verwendung von 3-Sulfenylmaleinimiden der Formel (IA)

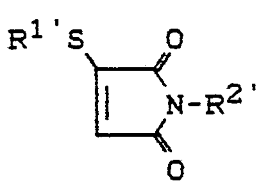

(IA)

in welcher
$R^{1'}$ für Alkyl, für gegebenenfalls ein-bis mehrfach substituiertes Cycloalkyl oder für gegebenenfalls ein-bis

mehrfach substituiertes Aralkyl steht und
$R^{2'}$ für gegebenenfalls ein-bis mehrfach substituiertes Alkyl, für gegebenenfalls ein-bis mehrfach substituiertes Cycloalkyl, für gegebenenfalls ein-bis mehrfach substituiertes Aryl oder für gegebenenfalls ein-bis mehrfach substituiertes Aralkyl steht, ausgenommen die Verbindung 3-Ethylsulfenyl-1-phenylmaleinimid, zur Bekämpfung von Schädlingen.

5. Verwendung von Verbindungen der Formel (IA) gemäß Anspruch 4, worin
$R^{1'}$ für geradkettiges oder verzweigtes Alkyl mit 1 bis 12 Kohlenstoffatomen, für gegebenenfalls ein-bis vierfach durch Alkyl mit 1 bis 4 Kohlenstoffatomen substituiertes Cycloalkyl mit 5 bis 10 Kohlenstoffatomen oder für gegebenenfalls ein-bis mehrfach, gleich oder verschieden substituiertes Aralkyl mit 6 bis 10 Kohlenstoffatomen im Arylteil und 1 bis 4 Kohlenstoffatomen im Alkylteil steht, wobei als Substituenten im Aryl genannt seien; Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl, Halogenalkoxy und Halogenalkylthio mit je 1 bis 3 Kohlenstoffatomen und 1 bis 5 Halogenatomen und Nitro;
$R^{2'}$ für geradkettiges oder verzweigtes Alkyl mit 1 bis 12 Kohlenstoffatomen, für gegebenenfalls ein-bis vierfach durch Alkyl mit 1 bis 4 Kohlenstoffatomen substituiertes Cycloalkyl mit 5 bis 10 Kohlenstoffatomen oder für gegebenenfalls ein-bis mehrfach, gleich oder verschieden substituiertes Aralkyl mit 6 bis 10 Kohlenstoffatomen im Arylteil und 1 bis 4 Kohlenstoffatomen im Alkylteil oder für gegebenenfalls ein-oder mehrfach, gleich oder verschieden substituiertes Aryl mit 6 bis 10 Kohlenstoffatomen steht, wobei als Substituenten im Arylteil des Aralkyls oder im Aryl selbst genannt seien: Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl, Halogenalkoxy und Halogenalkylthio mit je 1 bis 3 Kohlenstoffatomen und 1 bis 5 Halogenatomen und Nitro, ausgenommen ist die Verbindung 3-Ethylsulfenyl-1-phenylmaleinimid.

6. 3-Sulfenylmaleinimide der Formel (1B)

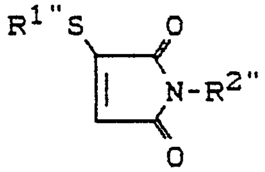

(IB)

in welcher
$R^{1''}$ für Alkyl, für gegebenenfalls substituiertes Cycloalkyl, für gegebenenfalls substituiertes Aralkyl steht und
$R^{2''}$ für gegebenenfalls substituiertes Cycloalkyl, für gegebenenfalls substituiertes Aralkyl oder für gegebenenfalls substituiertes Aryl steht, ausgenommen die Verbindungen, in denen $R^{1''}$ für Alkyl, und $R^{2''}$ für unsubstituiertes oder durch Halogen, Alkyl oder Alkoxy substituiertes Phenyl steht.

7. Verbindungen der Formel (IB) gemäß Anspruch 6, worin
$R^{1''}$ für geradkettiges oder verzweigtes Alkyl mit 1 bis 12 Kohlenstoffatomen, für gegebenenfalls ein-bis vierfach durch Alkyl mit 1 bis 4 Kohlenstoffatomen substituiertes Cycloalkyl mit 5 bis 10 Kohlenstoffatomen oder für gegebenenfalls ein-bis mehrfach, gleich oder verschieden substituiertes Aralkyl mit 6 bis 10 Kohlenstoffatomen im Arylteil und 1 bis 4 Kohlenstoffatomen im Alkylteil steht, wobei als Substituenten im Aryl genannt seien: Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl, Halogenalkoxy und Halogenalkylthio mit je 1 bis 3 Kohlenstoffatomen und 1 bis 5 Halogenatomen und Nitro;
$R^{2''}$ für gegebenenfalls ein-bis vierfach durch Alkyl mit 1 bis 4 Kohlenstoffatomen substituiertes Cycloalkyl mit 6 bis 10 Kohlenstoffatomen oder für gegebenenfalls ein-bis mehrfach, gleich oder verschieden substituiertes Aralkyl mit 6 bis 10 Kohlenstoffatomen im Arylteil und 1 bis 4 Kohlenstoffatomen im Alkylteil oder für gegebenenfalls ein-oder mehrfach, gleich oder verschieden substituiertes Aryl mit 6 bis 10 Kohlenstoffatomen steht, wobei als Substituenten im Arylteil des Aralkyls oder im Aryl selbst genannt seien: Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl, Halogenalkoxy und Halogenalkylthio mit je 1 bis 3 Kohlenstoffatomen und 1 bis 5 Halogenatomen und Nitro; ausgenommen die Verbindungen, in denen $R^{1''}$ für Alkyl, und $R^{2''}$ für unsubstituiertes oder durch Halogen, Alkyl oder Alkoxy substituiertes Phenyl steht.

8.Verwendung von Verbindungen der Formel (IB) gemäß den Ansprüchen 6 und 7 zur Bekämpfung von Schädlingen.

9. Schädlingsbekämpfungsmittel, gekennzeichnet durch einen Gehalt an mindestens einem 3-Sulfenylmaleinimid der Formeln (IA) oder (IB) gemäß den Ansprüchen 4 bis 7.

10. Verfahren zur Herstellung von Schädlingsbekämpfungsmitteln, dadurch gekennzeichnet, daß man 3-Sulfenylmaleinimide der Formel (IA) oder (IB) gemäß den Ansprüchen 4 bis 7 mit Streckmitteln und/oder oberflächenaktiven Mitteln vermischt.